(19) Europäisches Patentamt

European Patent Office

Office européen des brevets

(11) Publication number: **0 137 584**
**B1**

(12) **EUROPEAN PATENT SPECIFICATION**

(45) Date of publication of patent specification: **08.06.88**

(21) Application number: **84304175.7**

(22) Date of filing: **20.06.84**

(51) Int. Cl.⁴: **C 07 C 7/13, C 07 C 15/073**

(54) Separation of ethylbenzene from xylenes.

(30) Priority: **24.06.83 GB 8317209**

(43) Date of publication of application:
**17.04.85 Bulletin 85/16**

(45) Publication of the grant of the patent:
**08.06.88 Bulletin 88/23**

(84) Designated Contracting States:
**BE CH DE FR GB IT LI LU NL SE**

(56) References cited:
**EP-A-0 045 953**
**EP-A-0 083 202**
**FR-A-2 210 592**
**GB-A-1 550 391**
**US-A-3 558 732**
**US-A-3 855 333**
**US-A-4 031 156**
**US-A-4 368 347**

(73) Proprietor: **Exxon Research and Engineering
Company
P.O.Box 390 180 Park Avenue
Florham Park New Jersey 07932 (US)**

(72) Inventor: **MaGee, Ellington McFall
deceased (US)**

(74) Representative: **Northover, Robert Frank et al
ESSO Chemical Limited Esso Chemical Research
Centre P.O. Box 1
Abingdon Oxfordshire, OX13 6BB (GB)**

## Description

The separation of the various C₈ aromatic isomers from one another is well known in the chemical industry because of the usefulness of the different xylene isomers and ethylbenzene. Commercially, industry has concentrated its efforts on the recovery of para-xylene and among the well known recovery techniques are crystallization and and absorptive separation with molecular sieves. The separation and recovery of ethylbenzene is also desirable, e.g. as an intermediate in the manufacture of styrene, however, industry has had considerably less success in its recovery.

Various molecular sieves are disclosed as being selective for ethylbenzene, see U.S. Patents 3,943,182 and 4,175,099 where Group 1A exchanged Zeolite X is selective for ethylbenzene. However, the preponderance of literature discloses molecular sieves such as Types X and Y being selective for para-xylene over ethylbenzene. See U.S. Patents 3,558,730 and 4,069,172 which disclose separating para-xylene with a Type X or Y faujasite adsorbent containing barium and/or potassium. U.S. Patent 3,558,732 discloses separation of para-xylene with a Type X or Y zeolite with toluene as a desorbent, with benzene disclosed as being undesirable and inferior as a desorbent (still selective for para-xylene but less than toluene). U.S. Patents 3,626,020, 3,665,046, 3,663,638 and 3,686,343 disclose separation of para-xylene with various combinations of ion exchanges of Zeolite Y. Also of interest is UK Patent 1,307,264 which discloses para-xylene selectivity for a potassium Type Y molecular sieve. EP45953 describes a selective process for meta-xylene using a potassium zeolite Y and a toluene desorbent in which para-xylene is preferentially adsorbed with respect to ethylbenzene. The use of benzene as a desorbent or inert carrier is known and disclosed in a number of the above-cited patents relating to the recovery of para-xylene. However, the reversal of elution order with the use of benzene in the eluent and a high concentration of benzene on the sieve bed with a potassium Type Y molecular sieve is both unexpected and useful in the efficient separation of ethylbenzene.

## Summary of the invention

A process is disclosed for preferentially adsorbing and separating ethylbenzene from a feedstream containing the C₈ aromatic isomers of ethylbenzene and para-xylene by passing an eluent over a potassium Type Y molecular sieve, contacting the molecular sieve with the feedstream and desorbing the adsorbed ethylbenzene. The eluent has a concentration of benzene which reverses the elution order of para-xylene and ethylbenzene providing a sieve which preferentially adsorbs ethylbenzene over para-xylene.

## Background of the invention

According to the present invention it has unexpectedly been discovered that ethylbenzene is preferentially adsorbed from a feedstream admixture with at least one other C₈ aromatic including para-xylene when said mixture is contacted, under adsorption conditions, with a potassium Type-Y molecular sieve (i.e. containing predominantly potassium ions) and utilizing an eluent containing benzene. By "predominantly potassium ions" it is meant that 75—99+% (preferably at least 80%, more preferably at least 97%) of the sodium has been exchanged with potassium, preferably 80—99% and most preferably 85—98%. The adsorption may take place in either the liquid or vapour phase. The benzene is present on the sieve bed in high concentrations when the feedstream is passed over the sieve in order to obtain the preferential adsorption of ethylbenzene over para-xylene. The eluent is defined as containing the desorbent and optionally an inert carrier and used to desorb the adsorbed ethylbenzene. The adsorption of the ethylbenzene may typically take place at temperatures which vary broadly between 30° and 850°F ($-1$ and 455°C) and the pressure may typically vary between approximately 0.01 and 50 bar.

The eluent critically contains a concentration of benzene which unexpectedly reverses the adsorptive ability of the potassium exchange Type Y molecular sieve so that ethylbenzene is preferentially adsorbed over all other C₈ aromatics including para-xylene. With the eluent containing a high concentration of benzene the order in which the K—Y sieve preferentially adsorbs is ethylbenzene>para-xylene>ortho-xylene>meta-xylene thus allowing for the separation and recovery of ethylbenzene. The selectivity of ethylbenzene/para-xylene achieved by this process is generally 1.5 to 6. In adsorptive-separation processes an important factor that is used to determine the ability of a particular adsorbent to separate components of a feed is the selectivity of the adsorbent for one component as compared to another component. The selectivity as used throughout this specification is defined as the ratio of the two components of the adsorbed phase over the ratio of the same two components in the unadsorbed phase at equilibrium conditions. Expressed in equation form:

$$\text{Selectivity} = \frac{[\text{vol. percent C/vol. percent D}]_A}{[\text{vol. percent C/vol. percent D}]_U}$$

where C and D are two components of the feed represented in volume percent and the subscripts A and U represent the adsorbed and unadsorbed phase respectively. The equilibrium conditions as defined here were determined when the feed passing over a bed of adsorbent did not change composition after contacting the bed of adsorbent or in other words there was no net transfer of material occurring between the unadsorbed and adsorbed phases.

The benzene is present in the eluent and on the sieve in an amount effective to achieve the preferential adsorption of ethylbenzene over para-xylene, which amount can vary depending upon the amount of the feedstream. The concentration of benzene required in the eluent is at least 5%, preferably at least 40% by weight. The remainder of the eluent comprises an inert carrier.

Inert carriers which may be utilized include the following: Aromatics which are less tightly adsorbed than the xylenes, paraffins (both normal and iso), olefins, naphthenes, and mixtures of the above. The basic requirements for an inert carrier are: (1) a material liquid or gas at reaction conditions which does not react with the xylenes or molecular sieve at adsorption or desorption conditions; (2) which can easily be separated from the xylene product; and (3) does not effect the reversal of elution order. Examples ara paraffins from $C_2$ to $C_{15}$ (iso and normal), olefins from $C_2$ to $C_{18}$ (normal and branched), tetralin, decalin, and halogenated or oxygenated derivatives of the above. The preferred carrier for use with benzene in the eluent are low molecular weight paraffins. These are effective for unadsorbed xylene removal and can be easily separated from the ethylbenzene product, e.g. pentane, hexane or heptane.

In U.S. Patent 3,130,007 there is described the zeolite Type Y molecular sieve. The crystals of Zeolite Type Y are basically 3-dimensional framework of $SiO_4$ and $Al_2O_3$ tetrahedra cross-linked by the sharing of oxygen atoms. The valence of each tetrahedron containing aluminium is balanced by the presence in the aluminosilicate framework of a cation such as sodium ion. The void spaces in the framework are occupied by water molecules. Dehydration to effect the lowest order of hydration results in a crystal interlaced with channels of molecular dimension which offer high selectivity and surface area for the adsorption of particular molecules. The basic chemical formula for zeolite Y expressed in terms of moles of oxides may be written as $(0.9+0.2) Na_2O:Al_2O_3:W-SiO_2:XH_2O$ where W is a value greater than three and up to about 6 and X may be a value to about 9.

The ion exchange of the Type Y molecular sieve with potassium may be accomplished by any of the several different techniques known to those skilled in the art. Purely by way of non-limitative example, the following technique is given as a preferred method of ion exchanging the sodium for potassium.

The sodium form of the Type Y molecular sieve is treated hydrothermally with an aqueous solution of a potassium salt; chloride, nitrite or carbonate are preferred. The treatment involves agitation of the powdered Type Y sieve particles with the solution at temperatures from about ambient to 180° to 220°F (82° to 105°C). An excess of potassium ion is present above that required to replace the sodium ions ordinarily found in Type Y sieve stoichiometrically. A minimum of a 2-mole excess is preferred. After equilibration of the sieve with the exchange solution, usually in one or two hours, the excess of liquid is removed by filtration, decantation, or centrifugation. A second treatment is then carried out in the same manner, followed by a third and fourth if it is desired to obtain the maximum exchange of ions. A minimum of at least 2 treatments should be employed.

The liquid is removed and the solid particles are washed free of chloride ions, if potassium chloride is used as the exchange salt or source of potassium ions. The solid particles are then dried, and formed into pellets, extrudates, or any other desired form for use. They are calcined at temperatures of up to 1200°F (649°C), in air, prior to actual use in order to remove any excess water and to destroy any nitrate or carbonate ions which remain if these materials are used as exchange salts.

The feed stream for the separation process of the invention may be obtained from any of several sources. Briefly, a mixed $C_8$ aromatic feed stream containing the various xylene isomers and ethylbenzene may result from steam cracking of hydrocarbon oils, from coal tar sources, or from the reforming of hydrocarbon oils in petroleum process operations. In any case, a $C_8$ aromatics fraction is concentrated prior to separation, by distillation or other suitable means. In addition, the $C_8$ aromatic product or feed stream to an aromatic isomerization process may be used as feed for the process. A typical mixture to be treated by the process of the invention would have about 10 to 30 wt.% of para-xylene, 30 to 50 wt.% of meta-xylene, 10 to 30 wt.% of ortho-xylene and 10 to 30 wt.% ethylbenzene.

Any combination of ethylbenzene and other xylene isomers provides a suitable feed stream for the invention. The feed stream is passed over the predominantly potassium exchanged Type Y molecular sieve in either the liquid or vapor phase. The mixture is passed over the molecular sieve in the vapour phase generally at a rate of 0.01 to 10 weight feedstream/hr./weight of sieve. The adsorption technique of this invention can be cyclic, or a continuous process.

When a cyclic process is used, typical cycle times will vary between 2 and 480 minutes. Temperatures during adsorption may vary widely in the vapour phase. Acceptable limits would vary from 200—800°F (90—425°C), preferably 200—700°F (90—370°C) and most preferably 300—600°F (150—315°C). In the liquid phase the temperature may vary between 30 and 300°F (0—150°C) while preferred temperatures are between 70 and 210°F (20—100°C). The pressure may vary widely; acceptable limits are between 0.1 and 700 psig (0.007 and 48.3 bar), in both the liquid and vapor phases. For a vapor phase operation, a preferred pressure would be between 0.1 and 100 psig (0.007 and 6.9 bar). Pressure in the liquid phase should be sufficient to maintain liquid phase at the temperature utilised.

The eluent is introduced at a level which provides a eluent to feed by weight ratio of generally 2 to 20, preferably 2 to 6. The presence of the benzene in the eluent is critical to the preferential adsorption and recovery of ethylbenzene and is important to successful operation in the liquid phase because the eluent serves to sweep the interstices of the sieve while the feed stream is passed over it. Following ethylbenzene adsorption on the sieve, the ethylbenzene is desorbed with the eluent. The preferred method of using liquid phase adsorption is as follows:

1) Pass eluent over the sieve;

2) The mixed xylene and ethylbenzene feed stream is fed over the sieve in the liquid phase, with a raffinate stream being withdrawn which is depleted in ethylbenzene;

3) The adsorbent is desorbed by passing the eluent over the sieve producing an extract which contains the eluent and is rich in ethylbenzene; and

4) The extract containing ethylbenzene and eluent is then fractionated to separate the ethylbenzene.

Thus, when desorption takes place the product recovered is essentially ethylbenzene. Contaminants such as para-xylene, meta-xylene or ortho-xylene are minimized since they have been swept from the interstices and are substantially absent from the sieve.

It should be emphasized that although the preferred separation of the invention is that of ethylbenzene from at least one of the xylene isomers, the invention may be used to obtain any one of the $C_8$ aromatics used in pure form by proper use of adsorption and desorption techniques and stages. As mentioned, previously, there is a definite order which the various xylene isomers and ethylbenzene will be attracted to a predominantly potassium exchange Type Y molecular sieve when using a eluent containing a high concentration of benzene.

The desorption is by use of an eluent containing a desorbent and comprises benzene and may contain inert carriers as well. Desorption may be effected by passing the desorbent over the loaded bed. The eluent may be passed over the sieve in configurations such as countercurrently, concurrently or by cross flow in either a cyclic or continuous process. Typical temperatures during desorption will vary between 30 and 700°F, (0—370°C), preferably for liquid phase from 70 to 210°F (20—100°C). Pressure would vary between 0.1 and 700 psig, preferably 10 to 100 psig. For a cyclic process, typical cycles for desorption vary between 10 and 120 minutes, preferably 20 and 60 minutes.

Example I

In this example a mixed $C_8$ aromatic feed containing para-xylene, meta-xylene, ortho-xylene and ethylbenzene was treated with a potassium exchanged Type Y molecular sieve (K—Y faujasite) in order to separate the ethylbenzene.

The feed stream comprised the following amount of each component.

| Feed | Wt.% |
| --- | --- |
| p-xylene (PX) | 10 |
| m-xylene (MX) | 40 |
| ethylbenzene (EB) | 10 |
| ortho-xylene (OX) | 10 |
| nonane | 30 |

A liquid chromatograph was used to test the adsorbent-desorbent system. The liquid chromatograph had a column length X internal diameter of 91.4 cm×.41 cm and was packed with K—Y faujasite with a flow rate set at 0.25 ml/min and at a temperature of 25°C. After the eluent containing 50% by wt. of benzene and 50% by wt. of hexane had soaked the sieve, a sample of feedstream was injected into the liquid chromatographic bed, followed by desorption with the eluent.

The resultant elution order is shown in Figure I which demonstrates the preferential adsoption of ethylbenzene over the other $C_8$ aromatics obtained with an eluent containing benzene, allowing for the separation of ethylbenzene. The selectivity of EB/PX was 1.76 while the selectivity of EB/MX was 2.74.

Example II (Comparative)

As in Example I, a mixed $C_8$ aromatic feed containing para-xylene, meta-xylene, ortho-xylene and ethylbenzene was treated with a potassium exchanged Type Y molecular sieve (K—Y faujasite). In this example the eluent comprised 50 wt.% toluene and 50 wt.% hexane.

The feed stream comprised the following amount of each component:

| Feed | W.% |
| --- | --- |
| p-xylene (PX) | 15 |
| m-xylene (MX) | 40 |
| ethylbenzene (EB) | 15 |
| ortho-xylene (OX) | 15 |
| nonane | 15 |

A liquid chromatograph was used as in Example I to test the adsorbent-desorbent system. After the eluent containing 50% by wt. of toluene and 50% by wt. of hexane had soaked the sieve, a sample of feedstream was injected into the liquid chromatographic bed, followed by desorption with the eluent.

The resultant elution order is shown in Figure II which demonstrates the preferential adsorption of para-xylene over the other $C_8$ aromatics including ethylbenzene obtained with an eluent containing toluene. The selectivity of EB/PX was 0.36 (indicates selectivity for para-xylene over ethylbenzene), while the selectivity of EB/MX was 2.57.

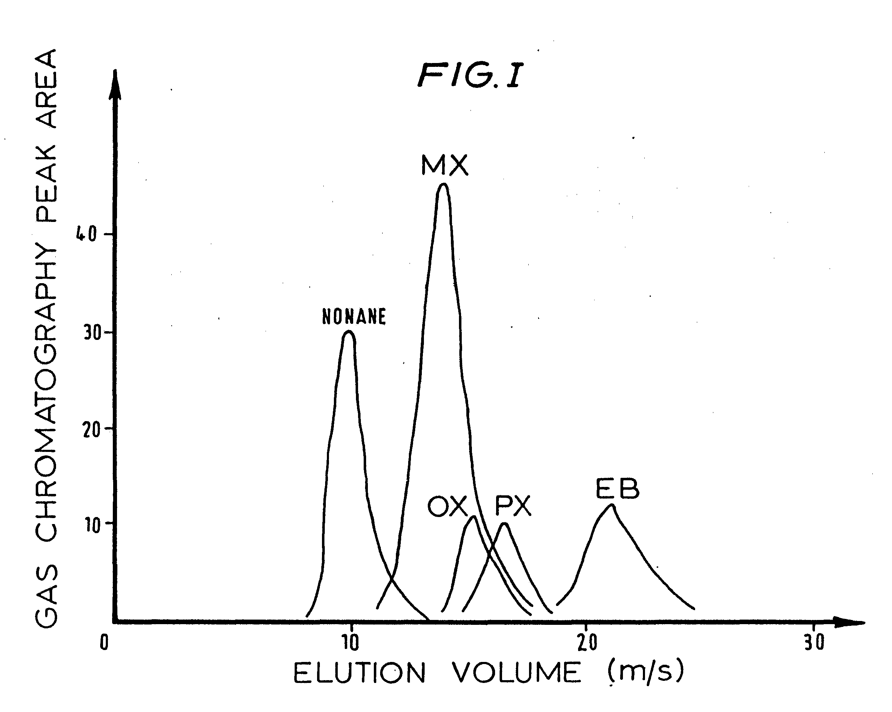

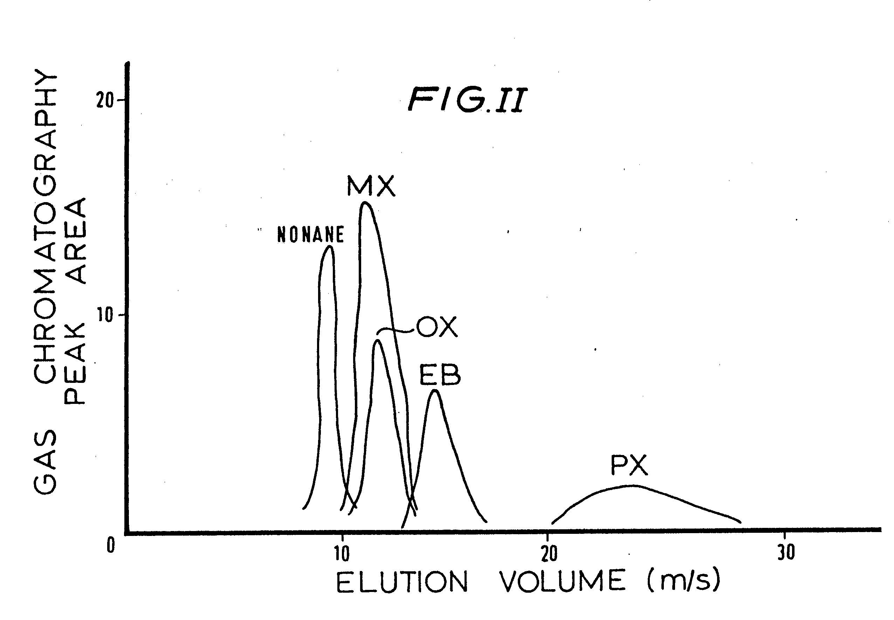

**Claims**

1. A process for preferentially adsorbing ethylbenzene from a feedstream comprising $C_8$ aroma-

tics including ethylbenzene and para-xylene comprising:

passing an eluent over a zeolite type Y molecular sieve in which at least 75% of sodium ions have been exchanged by potassium;

subsequently contacting the molecular sieve with the feedstream under adsorption conditions; and desorbing adsorbed $C_8$ aromatics from the molecular sieve with the eluent,

characterised in that the eluent contains benzene at a concentration of at least 5% by weight such that benzene is present on the sieve at a concentration sufficient to achieve the preferential adsorption of ethylbenzene in the molecular sieve soaked with eluent over all other $C_8$ aromatics including para-xylene, and that the adsorbed ethylbenzene is desorbed from the molecular sieve with the eluent.

2. A process as claimed in Claim 1 wherein the eluent contains benzene at a concentration of at least 40% by weight.

3. A process as claimed in Claim 1 or Claim 2 wherein the feedstream additionally contains meta-xylene and ortho-xylene.

4. A process as claimed in Claim 3 wherein the feedstream contains 10 to 30 wt.% of para-xylene, 30 to 50 wt.% of meta-xylene, 10 to 30 wt.% of ortho-xylene and 10 to 30 wt.% of ethylbenzene.

5. A process as claimed in any of Claims 1 to 4 wherein the process is carried out in the liquid phase at temperatures of 0 to 150°C.

6. A process as claimed in any of Claims 1 to 4 wherein the process is carried out in the vapour phase at temperatures of 90 to 425°C.

7. A process as claimed in any of Claims 1 to 6 wherein the eluent also contains an inert carrier from the group consisting of $C_2$—$C_{15}$ paraffins, $C_2$—$C_{18}$ olefins, tetralin, decalin, halogenated derivatives of the above and oxygenated derivatives of the above.

8. A process claimed in Claim 6 wherein the inert carrier is hexane.

9. A process as claimed in any of Claims 1 to 8 wherein the sieve has a mole ratio of $SiO_2$ to $Al_2O_3$ of 3 to 6.

## Patentansprüche

1. Verfahren zur vorzugsweisen Adsorption von Ethylbenzol aus einem $C_8$-Aromaten einschließlich Ethylbenzol und para-Xylol enthaltenden Ausgangsproduktstrom, bei dem ein Eluens über einen Zeolith Typ Y Molekularsieb geleitet wird, in dem zumindest 75% der Natriumionen durch Kaliumionen ausgetauscht worden sind, das Molekularsieb anschließend mit dem Ausgangsproduktstrom unter Adsorptionsbedingungen kontaktiert wird und adsorbierte $C_8$-Aromaten mit dem Eluens aus dem Molekularsieb desorbiert werden, dadurch gekennzeichnet, daß das Eluens Benzol in einer Konzentration von zumindest 5 Gew.% enthält, so daß das Benzol auf dem Sieb in einer ausreichenden Konzentration vorliegt, um die vorzugsweise Adsorption von Ethylbenzol an dem mit Eluens getränkten Molekularsieb gegen-

über allen anderen $C_8$-Aromaten einschließlich para-Xylol zu erzielen, und daß das adsorbierte Ethylbenzol aus dem Molekularsieb mit dem Eluens desorbiert wird.

2. Verfahren nach Anspruch 1, bei dem das Eluens Benzol in einer Konzentration von zumindest 40 Gew.% enthält.

3. Verfahren nach Anspruch 1 oder 2, in dem der Ausgangsproduktstrom zusätzlich meta-Xylol und ortho-Xylol enthält.

4. Verfahren nach Anspruch 3, in dem der Ausgangsproduktstrom 10 bis 30 Gew.% para-Xylol, 30 bis 50 Gew.% meta-Xylol, 10 bis 30 Gew.% ortho-Xylol und 10 bis 30 Gew.% Ethylbenzol enthält.

5. Verfahren nach einem der Ansprüche 1 bis 4, in dem das Verfahren in flüssiger Phase bei Temperaturen von 0 bis 150°C durchgeführt wird.

6. Verfahren nach einem der Ansprüche 1 bis 4, in dem das Verfahren in der Dampfphase bie Temperaturen von 90 bis 425°C durchgeführt wird.

7. Verfahren nach einem der Ansprüche 1 bis 6, in dem das Eluens ebenfalls einen inerten Träger aus der Gruppe von $C_2$—$C_{15}$-Paraffinen, $C_2$—$C_{18}$-Olefinen, Tetralin, Decalin und halogenierten und oxidierten Derivaten dieser Verbindungen enthält.

8. Verfahren nach Anspruch 6, in dem der inerte Träger Hexan ist.

9. Verfahren nach einem der Ansprüche 1 bis 8, in dem das Molekularsieb ein molares Verhältnis von $SiO_2$:$Al_2O_3$ von 3 bis 6 aufweist.

## Revendications

1. Procédé de séparation préférentielle par adsorption de l'éthylbenzène d'un courant d'alimentation renfermant des hydrocarbures en $C_8$, comprenant l'éthylbenzène et le para-xylène, consistant:

à faire passer un éluant sur un tamis moléculaire du type zéolite Y dans lequel au moins 75% des ions sodium ont été échangés contre des ions potassium; à mettre ensuite en contact le tamis moléculaire avec le courant d'alimentation dans des conditions d'adsorption; et à désorber du tamis moléculaire au moyen de l'éluant les hydrocarbures aromatiques en $C_8$ adsorbés, caractérisé en ce que l'éluant contient du benzène à une concentration d'au moins 5% en poids, de manière que le benzène soit présent sur le tamis à une concentration suffisante pour parvenir à l'adsorption préférentielle de l'éthylbenzène dans le tamis moléculaire imprégné de l'éluant, par rapport à tous les autres hydrocarbures aromatiques en $C_8$ comprenant le para-xylène, et en ce que l'éthylbenzène adsorbé est désorbé du tamis moléculaire au moyen de l'éluant.

2. Procédé suivant la revendication 1, dans lequel l'éluant contient du benzène à une concentration d'au moins 40% en poids.

3. Procédé suivant la revendication 1 ou la revendication 2, dans lequel le courant d'alimentation contient en outre du méta-xylène et de l'ortho-xylène.

4. Procédé suivant la revendication 3, dans lequel le courant d'alimentation contient 10 à 30% en poids de para-xylène, 30 à 50% en poids de méta-xylène, 10 à 30% en poids d'ortho-xylène et 10 à 30% en poids d'éthylbenzène.

5. Procédé suivant l'une quelconque des revendications 1 à 4, dans lequel le procédé est mis en oeuvre en phase liquide à des températures comprises dans l'intervalle de 0 à 150°C.

6. Procédé suivant l'une quelconque des revendications 1 à 4, dans lequel le procédé est mis en oeuvre en phase vapeur à des températures comprises dans l'intervalle de 90 à 425°C.

7. Procédé suivant l'une quelconque des revendications 1 à 6, dans lequel l'éluant contient également un support inerte choisi dans le groupe comprenant des paraffines en $C_2$ à $C_{15}$, des oléfines en $C_2$ à $C_{18}$, la tétraline, la décaline, des dérivés halogénés des composés précités et des dérivés oxygénés de composés précités.

8. Procédé suivant la revendication 6, dans lequel le support inerte est l'hexane.

9. Procédé suivant l'une quelconque des revendications 1 à 8, dans lequel le tamis possède un rapport molaire de $SiO_2$ à $Al_2O_3$ compris dans l'intervalle de 3 à 6.

FIG.I

FIG.II

GAS CHROMATOGRAPHY PEAK AREA

ELUTION VOLUME (m/s)

NONANE

MX

OX

EB

PX